# EUROPEAN PATENT APPLICATION

(11) **EP 2 700 430 A1**
(43) Date of publication of application: **26.02.2014**
(21) Application number: 12773496.0
(22) Date of filing: 18.04.2012
(51) Int. Cl.: A61N 5/06

(54) **CELL ACTIVATION DEVICE**

(30) Priority: 19.04.2011 JP 2011093524
(71) Applicant: Shima, Hiroki, Minoo-shi, Osaka 562-0046 (JP)
(72) Inventor: Shima, Hiroki, Minoo-shi, Osaka 562-0046 (JP)
(74) Representative: Horn Kleimann Waitzhofer
(86) International application number: PCT/JP2012/060410
(87) International publication number: WO 2012/144504

(57) **Abstract**

The purpose of the present invention is to solve issues with conventional hyperthermia treatment for cancer. The present invention provides a cell activation device that is capable of suppressing the progress of cancer and contributing to the irradiation of cancer cells. This cell activation device (100) activates the gene restoration mechanism naturally hound in cells and supports cancer treatment, by cutting infrared rays having a wavelength greater than 7.5 µm at which said rays works as heat rays and irradiating infrared rays having a wavelength which equals to or is smaller than 7.5 µm on an affected area containing cancer cells. An infrared irradiation therapy using wave length of no more than 7.5 µm is a gentle therapy that activates a latent function of human cells, such as activates the gene restoration mechanism. If a type that irradiates infrared rays without contact with the patient is used, cells can be activated while the patient is leading their normal life and without restricting physical freedom.

## Description

### Technical Field

This invention relates to a cell activation device for assisting cells activation. Especially, the cell activation device works on the cancer cells and peripheral cells for activating the gene restoration mechanism included in all cells based on human nature, for restraining the cancer cells growth, and extinguishing the generated cancer cells.

### Background Art

The present cancer treatment and therapy face many problems. If the cancer detection becomes late, a complete recovery is difficult, even if the cancer detection is success in early stage, the extirpation surgery is not always applied to the cancer depending on the lesion portion. In addition, side effect may be accompanied by the radiation therapy or chemotherapy. Even if cancer detection is success in early stage and the lesion portion is accessible to extirpate, the surgical treatment such as the extirpation is an invasive treatment method accompanying the injury of the human body.

In light of the current situation, the research of the cancer treatment and the development of the cancer treatment medical devices are actively performed, but the decisive cancer treatment method is not found.

Among the prior art, a prior art relating to the cancer treatment method using the thermotherapy is known as a non-invasive treatment (prior art 1 : JPA2003-126275). According to the prior art 1, the infrared ray which mainly works as a heating ray is irradiated to the lesion or whole body on the assumption that the cancer cell is weak with respect to high temperature. This thermotherapy requires enhancing the deep body temperature at the lesion part up to 41 °C to 42 °C.

The prior art 1 disclose the technology for enhancing the deep body temperature at the lesion part up to 41 °C to 42.5 °C by containing the patient in a capsule container which can contain his body except for his head air tightly, irradiating the infrared ray by the infrared irradiating means. The patient whole body except for his head is heated by the radiant heat of the infrared ray and the conductive heat of the air in the capsule container.

Patent prior art 1: JPA2003-126275

### Disclosure of the invention

### The problems to be solved

The conventional technology described in the prior art JPA2003-126275 has the following problem. According to the conventional technology described in the prior art JPA2003-126275, the blood temperature will be enhanced up to 45 °C 46 °C in order to enhance whole body temperature or the deep body temperature at the lesion part up to 41 °C to 42 °C. However, the conventional treatment term is limited up to one hour because a preliminary treatment such as an administration of heparin will be required in order to prevent blood from coagulating. The treated patient may be too damaged to standup for a few days due to a physical burden receiving in the conventional treatment.

In addition, according to the conventional technology described in the prior art 1, the radiant heat of the infrared ray is applied to the patient in order to enhance the body temperature up to 41 °C to 42 °C, the temperature of the skin surface will be enhanced more than 65 °C, the patient will suffer the low temperature burn during the long time irradiation. From this point of view, the conventional treatment term should be limited up to one hour.

On the contrary, the patient body temperature should be constrained lower to 43 °C within the physiological range for the normal cells. Therefore, it is difficult for achieving both the temperature of the normal cells are protected within the physiological range and the temperature of the cancer cells are enhanced up to 41 °C to 42 °C at the same time.

Therefore, it is an object of the present invention to provide a cell activation device which can activate the gene restoration mechanism included in all cells based on human nature and obtain the cell activation efficacies by irradiating the infrared ray.

### Means for solving the problems

Mr. Hiroki SHIMA, the inventor of the present invention keeps on working on researching the cancer treatment as a doctor, discovers the more effective cancer treatment than the conventional cancer treatment. Mr. Hiroki SHIMA's research is highly evaluated in the world, his thesis is published on the British science specialized magazine Nature (Nature Preceding : hdl : 10101/ npre. 2008.1980.1: Posted 17 Jun 2008).

The cell activation device of the present invention is developed by the inventor Mr. Hiroki SHIMA based on the newly obtained findings in this research. His invention can activate the gene restoration mechanism included in all cells based on human nature by irradiating the infrared ray by the different means from the conventional method.

In order to achieve the above-mentioned object, the present invention is a cell activation device for irradiating the infrared ray to the cells. It comprises a heat source, a temperature controller and an infrared ray irradiator. The radiation rays of wavelengths emitted by the infrared ray irradiator is controlled by the temperature controller, and only the infrared ray which wavelength equals to or is smaller than 7.5 µm (wavelength equals to or larger than that of the visible light and equals to or smaller than 7.5 µm) from the infrared ray irradiator is selectively utilized to irradiate onto the cells.

It is preferable that the cell activation device further comprises a filter which can pass only the infrared ray which wavelength equals to or is smaller than 7.5 µm is installed onto the surface of the infrared ray irradiator. The filter can cut-off the infrared ray which wavelength is larger than 7.5 µm mainly working as a heating ray, and passes only the infrared ray which wavelength equals to or is smaller than 7.5 µm for irradiating onto the cells.

In the above configuration, it is preferable that the fine holes are installed onto the infrared ray irradiator. If the diameter of the fine hole equals to or is smaller than 15 µm, the infrared ray irradiator does not irradiate the infrared ray which wavelength is larger than 7.5 µm mainly working as heating ray but irradiates the infrared ray which wavelength equals to or is smaller than 7.5 µm onto the cells.

In the above configuration, it is preferable that the depth of the fine holes of the infrared ray irradiator becomes larger than the diameter of the fine holes in order to obtain the radiation directivity on the infrared ray irradiator.

It is preferable that the cell activation device further comprises a first filter which is a hydrophilic filter having a quartz glass container filled with water and a second filter which can cut-off the infrared ray which wavelength is larger than 7.5 µm mainly working as heating ray.

In the above configuration, it is preferable that the temperature control part can maintain the temperature distribution of the heat source at a constant, and can maintain the surface temperature of the infrared ray irradiator within the particular temperature range.

### Effect of the invention

According to the above-mentioned configuration of the invention of a cell activation device, the infrared ray which works as a heating ray to the patient body is cut-off and the infrared ray which wavelength equals to or is smaller than 7.5 µm is selectively passed through for irradiating onto the cells in order to activate the gene restoration mechanism of each cell. By activating the gene restoration mechanism, the cell activation device can converts the cancer cells into the normal cells or can annihilate the cancer cells by activating the apoptosis process. The cell activation device can also constrain the conversion of the peripheral normal cells into cancer cells.

In addition, the cell activation device may be useful for the prophylaxis of other diseases in addition to the prophylaxis of cancer because the normal cells are activated.

### Brief description of the drawings

Fig.1 is a schematic drawing showing the experiment for verifying the effect on the human cancer cells by the infrared ray irradiation which wavelength equals to or is smaller than 7.5 µm by using the test sample of the cancer cells.
Fig.2 is a graphic chart showing a spectral reflectance of the special rubber material used in the present experiment.
Fig.3 is a graphic chart showing a spectral transmittance of the petri dish made from polystyrene, a spectral transmittance of the flexible disk made from polystyrene and a spectral transmittance of the silicon.
Fig.4 is a schematic chart showing an experiment result of the irradiation of the infrared ray which equals to or is smaller than 7.5 µm on the human cancer cells and the normal cells.
Fig.5 is a graphic chart showing a spectral transmittance of the water molecule and the carbon dioxide gas in the atmosphere for estimating a spectral infrared transmittance of the water molecule.
Fig.6 is a schematic drawing showing an effect on the human cancer cells on the condition that the infrared ray emitted from the human body is reflected to the human body without using the heat source.
Fig.7 is a graphic chart showing the result of the experiment shown in Fig.6.
Fig.8 is a schematic drawing showing a basic configuration of a cell activation device 100 of the present invention.
Fig.9 is a schematic drawing showing a basic configuration of a bed employing the cell activation device utilizing of the principle of the cell activation device of the present invention.
Fig.10 is a schematic drawing showing a gene restoration mechanism.
Fig.11 is a schematic drawing showing the effect on the reduction of the cancer cells by irradiating the infrared ray which wavelength equals to or is smaller than 7.5 µm.
Fig. 12 is a schematic drawing showing a basic configuration of a cell activation device 100a of Embodiment 2.

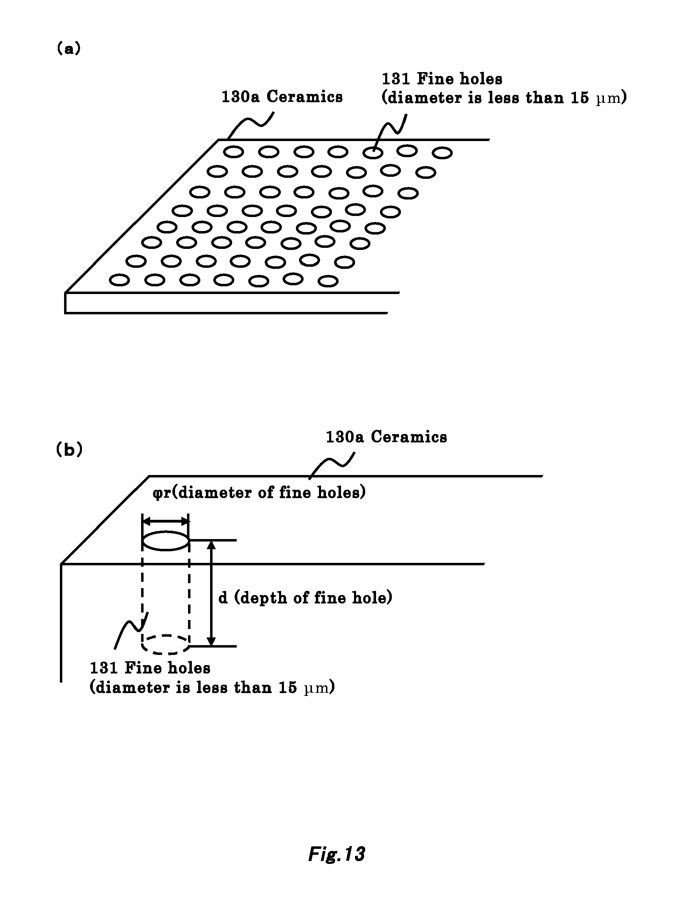

Fig. 13 is a schematic drawing showing the relationship between the depth (d) and the diameter (r) of the fine holes and the directivity of the reflected infrared ray.
Fig. 14 is a schematic drawing showing a water filter filled with water in the quartz glass container.

### The reference number in the Figs.

- 10: denotes a test sample
- 20: denotes culture solution
- 30: denotes an infrared ray irradiator
- 40: denotes a petri dish
- 100: denotes a cell activation device
- 110: denotes a heat source
- 120: denotes an infrared ray irradiator
- 130: denotes an infrared ray wavelength controller (optical filter)
- 130a: denotes a ceramics
- 130b: denotes a water filter filled with water in the quartz glass container
- 140: denotes a temperature controller

### Detailed description of the preferred embodiment

Some embodiments of a cell activation device according to the present invention are described below with reference to the relevant drawing. Needless to add, the claims of the present invention include but are not limited to the configuration and numerical value shown in the following embodiments.

### Embodiment 1

First, the experiment which shows the basic principle of the cell activation device 100 is described below.

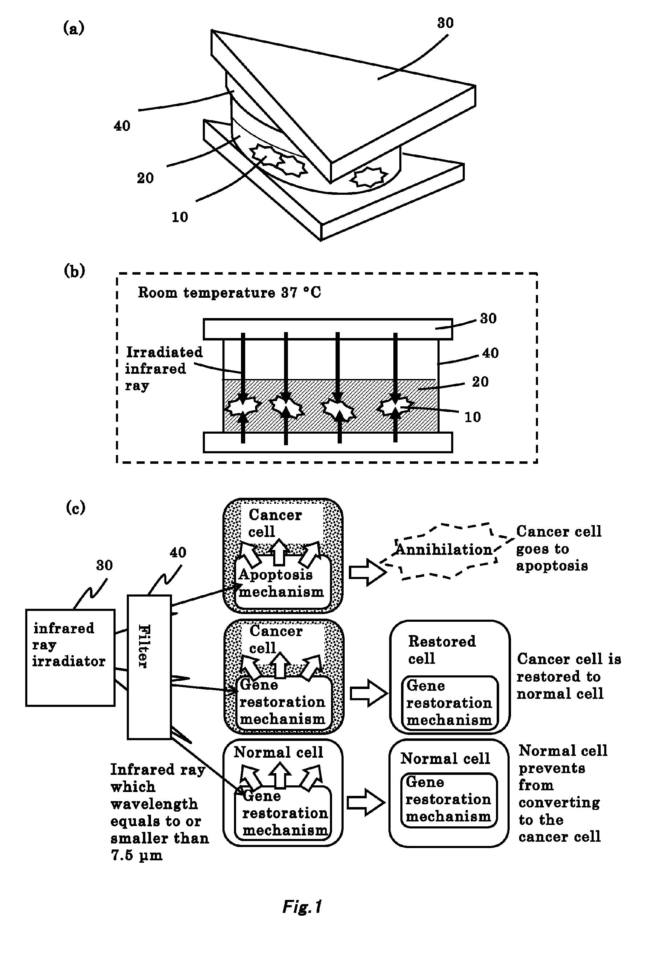

Fig.1 is a schematic drawing showing the experiment for verifying the effect on the human cancer cells by the infrared ray irradiation which wavelength equals to or is smaller than 7.5 µm by using the test sample of the cancer cells.

As shown in Fig.1 (a), a test sample 10 and culture solution 20 are put on the petri dish 40, an infrared ray irradiator material 30a is installed on the petri dish 40 and an infrared ray irradiator material 30b is installed beneath the petri dish 40.

Fig.1 (b) is a schematic drawing showing the cross-sectional view of the each component shown in Fig.1 (a).

The test sample 10 is a tissue including human cancer cells. As an example, the human derived prostatic cancer cells are prepared.

The popular culture solution may be employed as the culture solution 20. As an example, amino acid solution including the fetal calf serum, L-glutamine and penicillin is prepared.

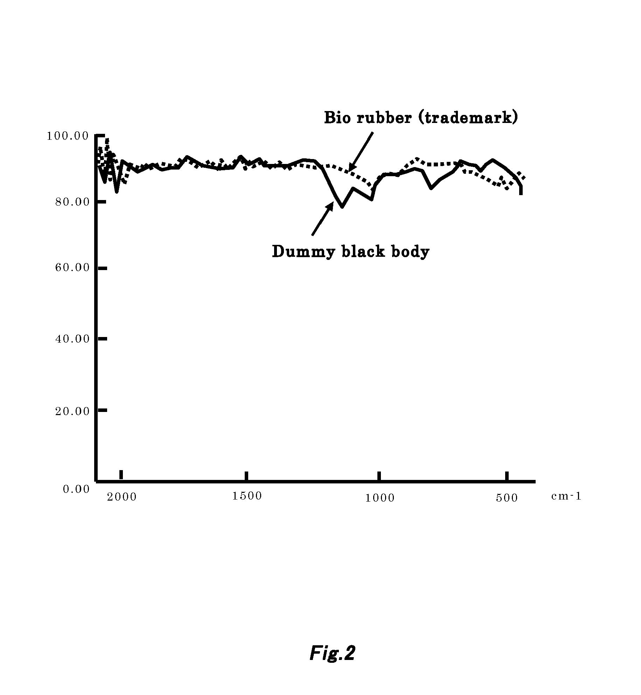

The infrared ray irradiator 30a and 30b are the material which can irradiate the infrared ray. In this experiment, a rubber material (Bio rubber (trademark): made by Yamamoto Corporation Co., Ltd.) is used. It has fine holes on the surface of the rubber plate for reflecting the infrared ray emitted from the human body. Fig.2 is a graphic chart showing a spectral reflectance of the special rubber material used in the present experiment. As shown in Fig.2, the spectral characteristics of the infrared ray irradiator 30a and 30b is flat constant spectral characteristics for 1 to 20 µm range.

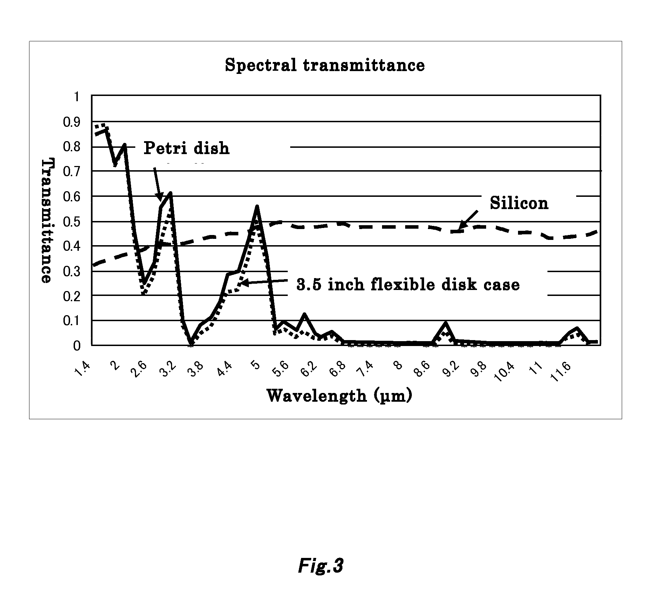

The petri dish 40 made from polystyrene is used. The spectral transmittance of the petri dish 40 is shown as Fig.3. As shown in Fig.3, most of the infrared ray which wavelength equals to or is larger than 5 µm is cut-off, especially the transmittance of the infrared ray which wavelength is larger than 7.5 µm is zero. In Fig.3, a spectral transmittance of the petri dish used in this experiment and a spectral transmittance of the flexible disk used in this experiment are shown together. A spectral transmittance of the silicon (Si) is also shown together in Fig.3. The spectral transmittance of the silicon (Si) is flat constant spectral characteristics. The filter including silicon material is described later on.

The petri dish 40 works as a filter in the experiment shown in Fig.1. The infrared ray which wavelength is larger than 7.5 µm among the infrared ray emitted from the infrared ray irradiator 30a and 30b is selectively cut-off by the petri dish 40. Therefore, only the infrared ray which wavelength equals to or is smaller than 7.5 µm can be irradiated to the test sample 10.

The experiment apparatus shown in Fig.1 is set in an incubator, which means a culture solution installed in a heat-retaining storage, the temperature is maintained in 37 °C and the concentration of carbon dioxide gas is maintained in 5 % the same as those of popular cultivation experiment.

After the experiment is started with the infrared ray irradiator 30a and 30b, the temperature of the culture solution 20 in the petri dish 40 became a little bit higher up to 37.68 °C because of the infrared ray emitted from the infrared ray irradiator 30a and 30b in addition to the heat derived from the cell proliferation. On the contrary, after the experiment is started without the infrared ray irradiator 30a and 30b, the temperature of the culture solution 20 in the petri dish 40 became 37.32 °C even the heat derived from the cell proliferation is generated.

The experiment term is 28 days. During the experiment term, the temperature of the incubator is always maintained to 37 °C. The temperature of the test sample 10 reaches 37.68 °C and the cultivation of the test sample 10 is going on for 21 days. After 21 days cultivation, surviving cancer cells is collected and the number of the surviving cancer cells is counted up, and then each cancer cells are put back in the cultivation solution 20 in the petri dish 40 again. Further 7 days cultivation is going on, and then the cancer cells are collected and the number of the surviving cancer cells is counted up on the 29th day. The number of the surviving cancer cells is counted by XTT assay kit. As the XTT assay kit, the Cell Proliferation Kit II (Roche Diagnostics, Mannheim, Germany) is used.

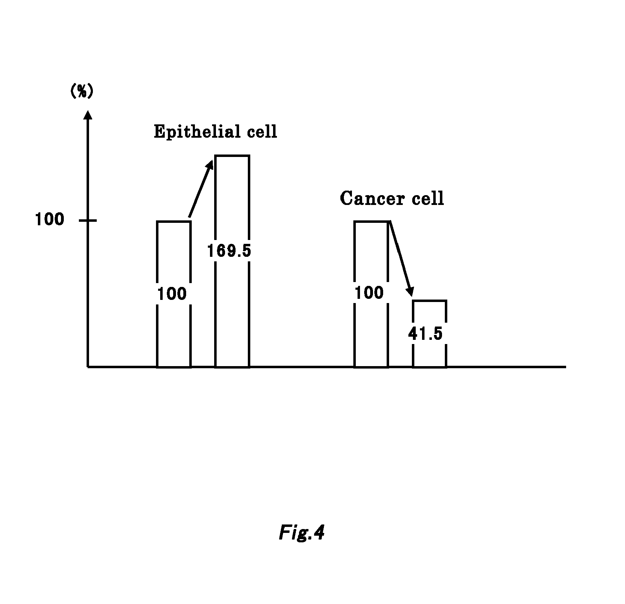

The experiment result is shown in Fig.4. The schematic chart described the increasing rate (%) of the number of the surviving cancer cells on the 29th day on the basis of the number of the 21st day.

As the experiment result, if the experiment started with the infrared ray irradiator 30a and 30b, the number of the cancer cells is decreased 58.5 % down to 41.5 % comparing with the case if the experiment started without the infrared ray irradiator 30a and 30b. Regarding the normal cells (human epithelial cells), if the experiment started with the infrared ray irradiator 30a and 30b, the number of the normal cells is increased 69.5 % up to 169.5 % comparing with the case if the experiment started without the infrared ray irradiator 30a and 30b.

There should be two possible reasons for annihilating 58.5 % cancer cells. The first reason is that when the temperature of the cultivate solution 20 reaches 37.68 °C, the apoptosis mechanism is activated in the cancer cells and the cancer cells annihilates itself as shown in the upper portion of Fig.1 (c). The second reason is that the gene restoration mechanism included in all cells is activated in the cancer cells and the speed of the cell proliferation become small because the cancer cells are converted into the normal cells as shown in the middle portion of Fig.1 (c). As shown above, it is understand from the experiment that the gene restoration mechanism is activated and the number of the cancer cells is decreased, and the cancer cells are converted into the normal cells.

Regarding the normal cells (human epithelial cells), the normal cells are activated for cell proliferation and the gene restoration mechanism is also activated. On the contrary, the apoptosis mechanism is not activated in the normal cells. If the gene is damaged in the normal cells, the damaged gene can be restored before the apoptosis mechanism is activated.

The reason for the activation of the gene restoration mechanism is not simply because of the temperature up to 37.68 °C but because of the infrared ray emitted from the cells accompanying the cell proliferation is reflected by the infrared ray irradiator 30a and 30b, and the infrared ray which wavelength is larger than 7.5 µm is cut-off by the material of the petri dish 40 and the infrared ray which wavelength equals to or is smaller than 7.5 µm is irradiated to the depth portion of the test sample 10.

Hereinafter, the possibility that the infrared ray which wavelength equals to or is smaller than 7.5 µm can reach to the depth portion of the sample 10 is described. As described above, in the experiment apparatus shown in Fig.1, the petri dish 40 works as a filter for cutting of the infrared ray which wavelength is larger than 7.5 µm and passing through the infrared ray which wavelength equals to or smaller than 7.5 µm among the infrared ray which is emitted from the infrared ray irradiator 30a and 30b at the 37.68 °C.

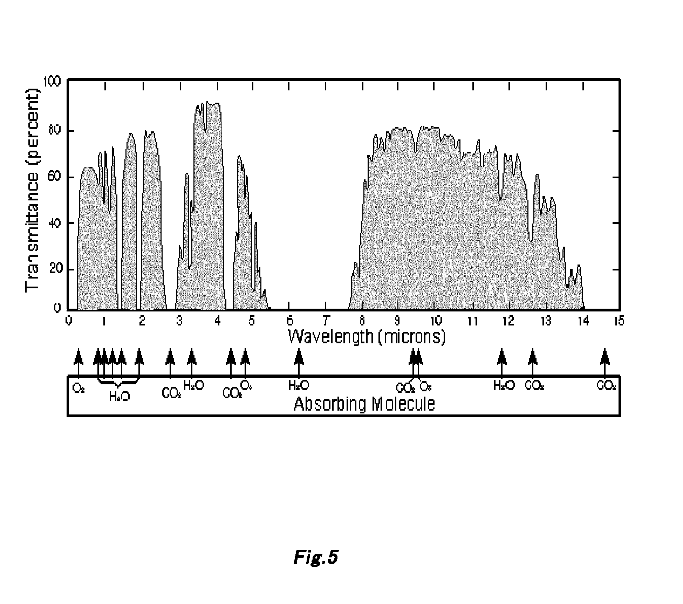

The infrared ray which wavelength equals to or smaller than 7.5 µm passing the petri dish 40 lid passes through the atmosphere including 5 % concentration of carbon dioxide gas and further passes through the cultivation solution, cell wall and cytoplasm. As shown in Fig.5, the infrared ray which wavelength equals to or smaller than 7.5 µm can pass through the carbon dioxide gas judging from the spectral transmittance of the carbon dioxide gas and its absorption spectrum.

There are a lot of fine holes which diameter is 2 to 7 µm in the cell such as the fine holes on the surface of the mitochondria. Therefore, the infrared ray which wavelength is smaller than the diameter of these fine holes can enter the inside of the cytoplasm via the cell wall. Regarding the cytoplasm, about 90 % of the human body is composed of water. As shown in Fig.5, the infrared ray which wavelength equals to or smaller than 7.5 µm can pass through the water judging from the spectral transmittance of the water molecule.

Considering the experiment result, the infrared ray which wavelength equals to or smaller than 7.5 µm can reach depth portion of the test sample 10 and activate the gene restoration mechanism and the apoptosis mechanism in the cancer cells in the test sample 10, and the number of the cancer cells is decreased.

Next, the characteristics of the bio rubber (trademark) used as the material of the infrared ray reflection.

The bio rubber (trademark) used as the material of the infrared ray reflection in the experiment is normally used as the material for body suits having the characteristics of the infrared ray reflection. In short, bio rubber (trademark) is used as the suits material for wet suits to put on the human body for warming the user body by reflecting the infrared ray emitted from the human body back to the human body. Therefore, if the user puts bio rubber (trademark) suits on the body as usual, only the infrared ray emitted from human body at the temperature of 36.5 °C is returned to the user body. The strength of the required infrared ray wavelength radiation is deficient.

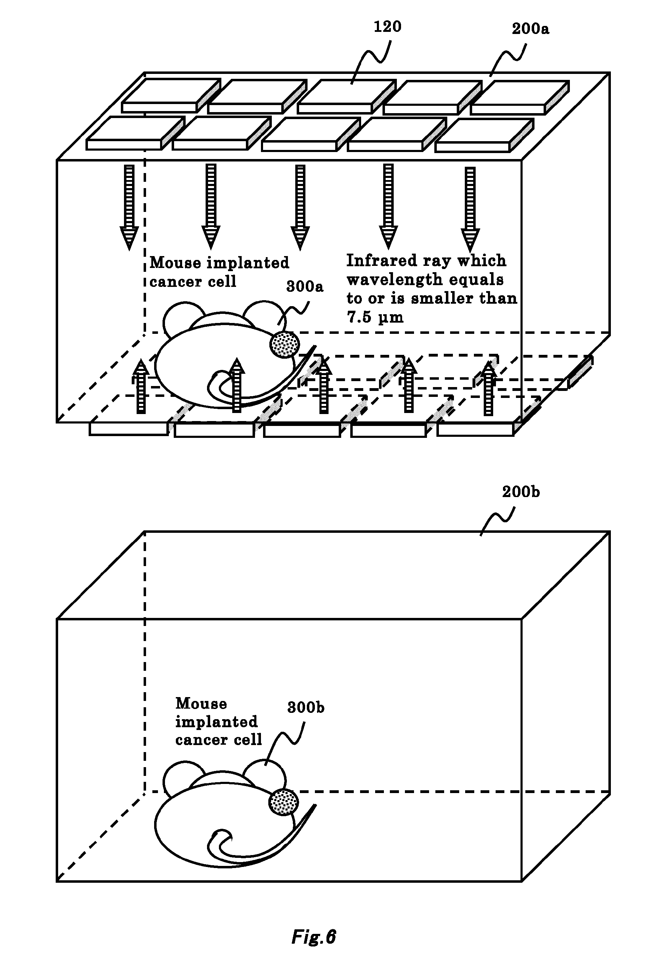

Fig.6 is a schematic drawing showing an effect on the human cancer cells on the condition that the infrared ray emitted from the human body is reflected to the human body by using the bio rubber (trademark) without using the heat source. A node mouth is used in this experiment. The filter for adjusting the infrared ray wavelength is not installed in this experiment. In Fig.6, the bio rubber (trademark) is drawn only on the upper side of the gauge, and others on the other surfaces are omitted here. However, all surfaces such as the side surface and bottom surface are covered by bio rubber (trademark) plates.

As shown in Fig.6, test mouse 300a and 300b onto which the cancer cells are implanted are put in gauge 200a and 200b respectively. All surfaces of the upper gauge 200a are covered by the bio rubber (trademark) as the infrared ray irradiator 110. The lower gauge 200b is not covered by the bio rubber (trademark) at all.

3 millions of human prostate cancer cells are implanted to the hypodermic tissue of the back skin of the node mouse 300a and 300b respectively and the growth of the cancer cells are observed. Mouse does not have T-cell, so there is no rejective reaction caused by the cancer cells. The infrared ray which wavelength equals to or smaller than 7.5 µm is always irradiated to the node mouse 300a in the gauge 200a, but the infrared ray which wavelength equals to or smaller than 7.5 µm is not always irradiated to the node mouse 300b in the gauge 200b. Other breeding condition is the same.

The mouse 300a and the mouse 300b are emitting the infrared ray corresponding to its body temperature. There is the infrared ray irradiator 110 is installed in the gauge 200a, so the emitted infrared ray is reflected to from the infrared ray irradiator 110 to the mouse 300a.

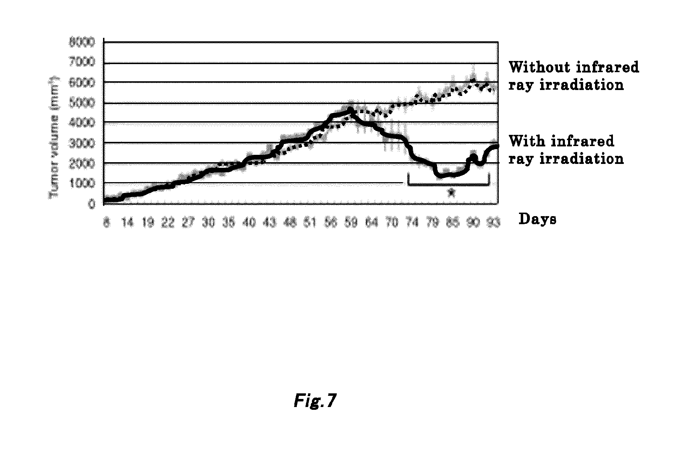

The temperature in the gauge is maintained as 23 °C and the humidity in the gauge is maintained as 40 %. The experiment is executed for 93 days. The experiment result is shown in Fig.7. If the mouse is bred in a natural condition, the number of the human cancer cells keeps on increasing during the experiment term because the cancer cells proliferate in the mouse body freely. As shown in Fig.7, the number of the cancer cells in the mouse 300b bred in the gauge 200b in which the infrared ray is not irradiated back to the mouse is increased to the last. On the contrary, the number of the cancer cells in the mouse 300a bred in the gauge 200a in which the infrared ray is irradiated back to the mouse is increased from the beginning up 70 days, but turned to decreased over 70 days. This result indicates the effect on the restraint of the proliferation of the cancer cells by irradiating the infrared ray by reflecting the emitted infrared ray from the body though requiring a long period of time for 70 days.

However, as shown in Fig.4, the effect on the restraint of the proliferation of the cancer cells by irradiating the infrared ray which wavelength equals to or is smaller than 7.5 µm is verified enough. If the infrared ray emitted from the mouse is reflected by the bio rubber (trademark) without any heat source as shown in Fig.6, the irradiation intensity of the effective infrared ray which wavelength is smaller than 7.5 µm is not enough. Therefore, it takes 70 days to show the distinguishable effect on the restraint of the proliferation of the cancer cells.

The reason for the slow-acting of the effect on the restraint of the proliferation of the cancer cells is the deficiency of the infrared ray which wavelength is smaller than 7.5 µm by utilizing only the infrared ray emitted from the human body (mouse) by putting the bio rubber (trademark) as the infrared ray irradiator 110 on the human body (mouse).

The cell activation device 100 of the present invention includes a heat source for enhancing the irradiation intensity of the effective infrared ray which wavelength is smaller than 7.5 µm in order to obtain the distinguishable effect on the restraint of the proliferation of the cancer cells earlier.

The cell activation device 100 of the present invention employs the infrared ray reflection material for irradiating the infrared ray which wavelength is smaller than 7.5 µm, the gene restoration mechanism is activated in the normal cells and the cancer cells by irradiating the infrared ray to the lesion.

The cell activation device 100 of the present invention is provided as the non-contact style device. The conventional bio rubber is provided as a wear material for contacting human body. The patient cannot help wearing the bio rubber (trademark) for 24 hours if the infrared ray irradiation is required constantly, but it is inconvenient for the patient because the freedom of behavior of the patient is deprived

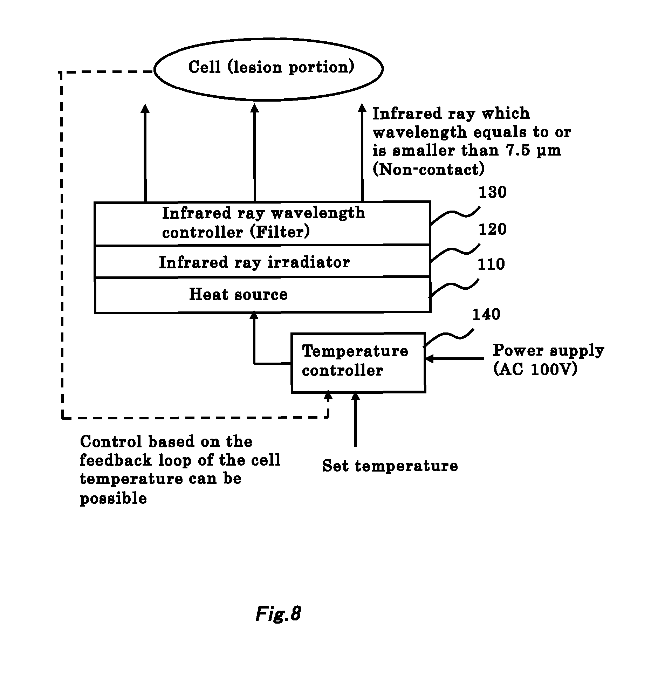

Fig.8 is a schematic drawing showing a basic configuration of a cell activation device 100 of the present invention.

As shown in Fig.8, the basic configuration of the cell activation device 100 can irradiate the infrared ray which wavelength is smaller than 7.5 µm and it is a non-contact style device apart from the lesion of the patient for irradiating the infrared ray which wavelength is smaller than 7.5 µm from the outside.

As shown in Fig.8, the cell activation device 100 includes the infrared ray irradiator 110, the heat source 120, the infrared ray wavelength controller 130 and the temperature controller 140.

The infrared ray irradiator 110 is a material irradiating the infrared ray depending on the temperature of the material. The material is not limited if the material can emit the infrared ray proportional to the temperature of the object. For example, the bio rubber (trademark), the ceramics such as tile, the metal material such as the bronze plate and the aluminum plate can be employed as this material.

The heat source 120 generates and supplies heat to the infrared ray irradiator 110. The heat source 120 is not limited if it can control the heat amount and temperature. For example, the device which can control the electric current and voltage such as plate heat device can be employed.

The infrared ray wavelength controller 130 cuts off the infrared ray which wavelength is larger than 7.5 µm working as heating ray and passes through the infrared ray which wavelength equals to or is smaller than 7.5 µm. In this example, it is an optical filter. As the material for the filter, the bio rubber (trademark), water, polystyrene resin can be applicable. Other material beside water and polystyrene resin can be applicable on the condition that it has characteristics for cutting off the infrared ray which wavelength is larger than 7.5 µm and passing through the infrared ray which wavelength equals to or is smaller than 7.5 µm.

If the filter should be cooled in order to avoid the temperature becoming too high by the heat, various cooling device such as the heat dissipation through heat conduction using heat sink, the air cooling or the liquid cooling using heat medium and the cooling device using coolant can be applicable.

The temperature controller 140 is a controller for fine controlling the temperature of the heat source 120.

There are varieties of controlling processes for the temperature controller. Such these controlling processes are kind of conventional technology, it is not limited. In this example, the temperature controller 140 includes the voltage controller device for adjusting the temperature by utilizing the input voltage (AC 100V).

By using those elements, the cell activation device 100 of the present invention can cut off the infrared ray which wavelength is larger than 7.5 µm and pass through the infrared ray which wavelength equals to or is smaller than 7.5 µm.

As shown in Fig.8, it is possible to achieve the non-contact device which can irradiate the required amount of the infrared ray for activating the cell. This non-contact device can cut off the infrared ray which wavelength is larger than 7.5 µm working as heat ray and passes through the infrared ray which wavelength equals to or is smaller than 7.5 µm for activating the apoptosis mechanism for annihilating the cancer cells.

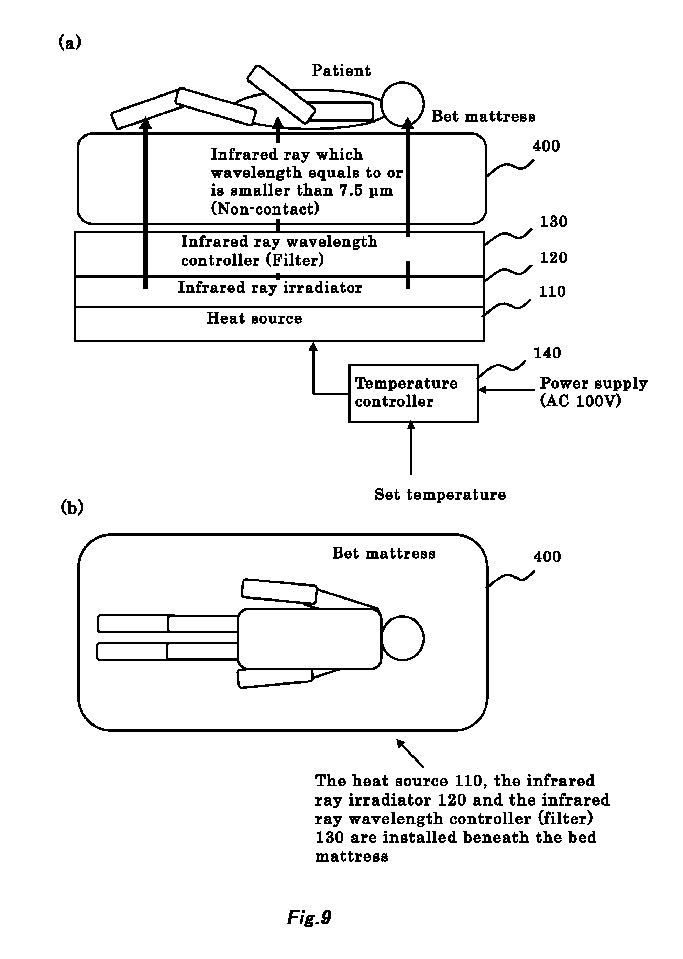

Fig.9 is a schematic drawing showing a basic configuration of a cell activation bed employing the cell activation device. As shown in Fig.9, the heat source, the infrared ray irradiating object and the filter are installed beneath the bed. The patient is always exposed to the infrared ray which wavelength equals to or is smaller than 7.5 µm irradiated under the bed if the patient laid down the bed. The heat radiation countermeasure should be conducted to avoid heat retention beneath the bed.

The bed shown in Fig.9, the bed includes the heat source 120, the infrared ray irradiation object 110 and the filter 130 beneath the bed. Other configuration of the non-contact device can be possible. For example, a ceiling light includes the heat source 120, the infrared ray irradiation object 110 and the filter 130 in order to irradiate the infrared ray which wavelength equals to or is smaller than 7.5 µm over the head of the patient.

Next, the principle to attain cancer treatment closer to natural healing to the cancer cells by activating the gene restoration mechanism is described.

There are still many uncertainties as to the cause of the generation of the cancer, but the one cause is the damage on the gene injured for somewhat reason. The gene restoration mechanism is included in all cells based on the human nature to restore the damaged gene to the normal gene. When the gene is damaged in each cell, the damaged gene is restored by the gene restoration mechanism.

However, when the gene restoration cannot catch up with the gene deterioration by the decrease of the gene restoration rete accompanying the cell aging or the increase of the gene damage rate due to the environmental factor, the cell is classified into 3 patterns. First pattern is the cell which goes into the dormant status of being irreversible called the aging (aging of cell). Second pattern is the cell which kills itself called the apoptosis or the programmed cell death. Third pattern is the cell which converts into cancer. As a result, some cells convert into cancers unless the damaged gene is not restored to the normal gene. If the cell is converted into the cancer cell, the cancer cell will proliferate as a cancer cell.

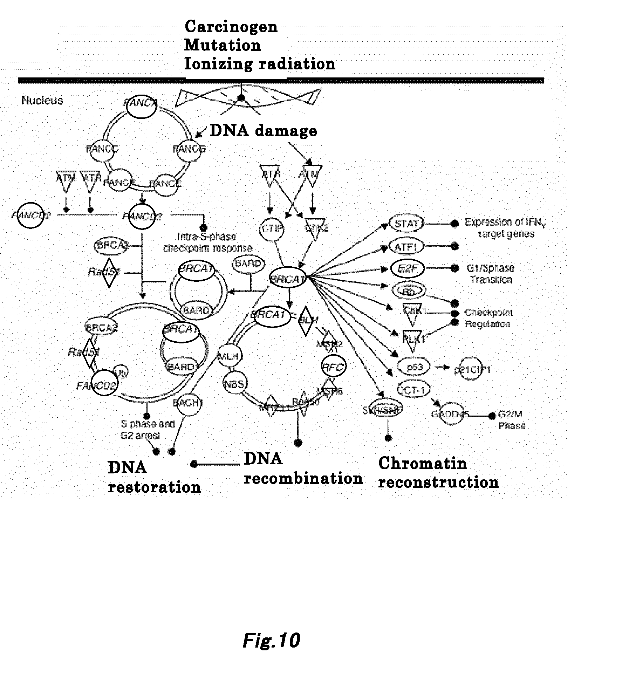

Fig. 10 is a schematic drawing showing a gene restoration mechanism. It shows the restoration cycle for restoring the damaged gene to the normal gene in each cell. If the gene restoration mechanism is activated, the damaged gene in the cancer cell is restored to the normal gene and normal cell.

The cancerous growth is caused by repeating the cell proliferation for generating new cancer cell because the gene restoration mechanism in the cell does not work normally.

The cell activation device 100 can converts the damaged cancer cells into the normal cells by activating the gene restoration mechanism included in all cells based on human nature and can annihilate the damaged cancer cells by promoting the apoptosis in the cancer cells. In addition the cell activation device 100 can protect the conversion of the normal cells around the cancer cells into the newly cancer cells

Next, the effect of the cancer cells by irradiating the infrared ray which wavelength equals to or is smaller than 7.5 µm is verified as follows.

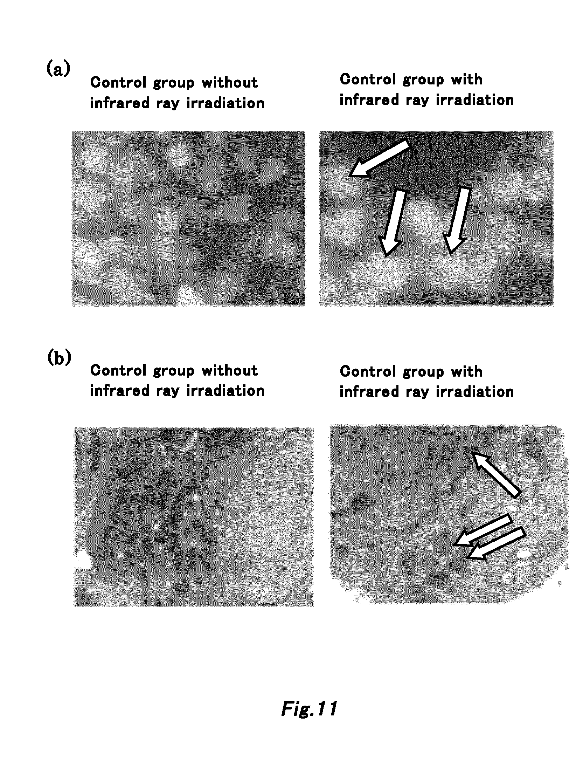

The human cancer cells are cultivated with irradiation of the infrared ray which wavelength equals to or is smaller than 7.5 µm for 28 days and the human cancer cells is put into the petri dish having 35mm diameter on 29th day and observed. As shown in Fig. 11 (a), the nucleus of the cancer cells are dyed with the propidium iodide followed by dying of TUNEL (Termi-nal deoxynucleotidyl transferase (TdT)-mediated deoxyuridine triphosphate (dUTP)-digoxigenin nick end labeling (TUNEL)), and the apoptosis working in the processed cancer cells are observed by the fluorescence microscope. The apoptosis is observed in the cell (indicated by arrow) among cancer cells exposed to the infrared ray which wavelength equals to or is smaller than 7.5 µm.

As shown in Fig.11 (b), the processed cancer cells are observed at magnification of 10,000 times by the electron microscope. The apoptosis characteristics such as the swelling of the mitochondria, the blurring of the crista, the swelling and the corrugating of the nuclear membrane and the vacuolation of the cytoplasm are observed in the cancer cell in the experimental group with irradiating the infrared ray which wavelength equals to or is smaller than 7.5 µm.

As shown above, the gene restoration mechanism in each cancer cell is activated by irradiating the infrared ray which wavelength equals to or is smaller than 7.5 µm. The effect on converting the cancer cells into the normal cells, annihilating the cancer cells, and preventing the normal cells around the cancer cells from converting into the cancer cells are verified through the above-mentioned experiment.

In the above experiment, the temperature of the object is discussed to express the infrared ray wavelength. It is know that the energy amount obtained by the wavelength of the infrared ray and the frequency is proportion to the temperature of the object.

### Embodiment 2

As shown in Fig.8, the configuration shown in Embodiment 1 employs the filter as an element for cutting off the infrared ray which wavelength equals to or is smaller than 7.5 µm. However, the element for cutting off the infrared ray which wavelength equals to or is smaller than 7.5 µm is not limited to the filter. Other element can be employed instead of the optical filter. The embodiment 2 of the configuration of the cell activation device employing other element instead of the optical filter is described below.

The plate object having fine holes on the surface can be employed as an alternative of the optical filter. The object having fine holes which diameter is as twice as the wavelength of the infrared ray to be cut off can be an alternative element instead. For example, the resin plate object, the ceramics plate and other material can be employed as an alternative object. The object having fine holes on its surface has characteristics that the ray which wavelength larger than 1/2 of the diameter of the fine holes is cut off. If the diameter of the fine holes is 15 µm, the object has the same characteristics as the filter which cut-off wavelength is 7.5 µm, so the alternative object can cut off the infrared ray which wavelength is larger than 7.5 µm.

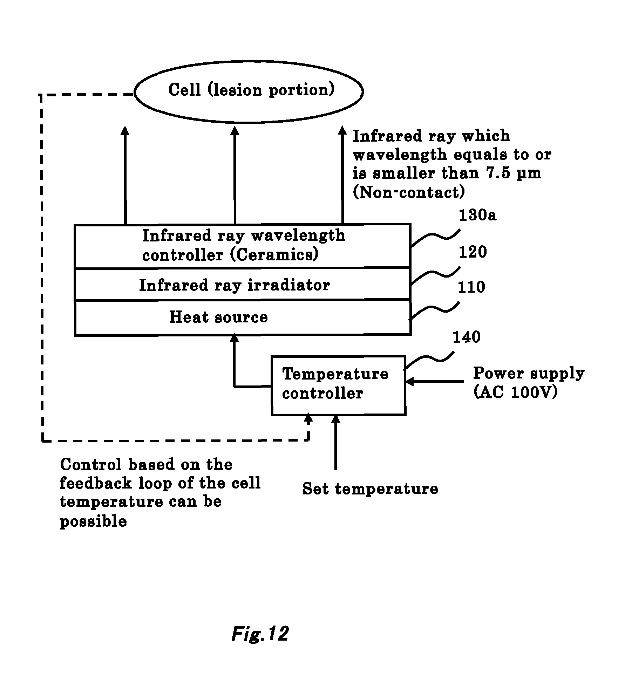

Fig. 12 is a schematic drawing showing a basic configuration of a cell activation device 100a of Embodiment 2.

As shown in Fig. 12, the cell activation device 100a of Embodiment 2 includes a ceramics 130a such as ceramic plate having a high emissivity in the infrared ray, and the ceramics 130a has fine holes which diameter is 15 µm on its surface. Other plate object except for the ceramics 130a can be employed if the fine holes can be installed on the surface.

In the configuration shown in Fig.12, the infrared ray which wavelength equals to or is smaller than 7.5 µm is passed through the plate object, and the infrared ray which wavelength is larger than 7.5 µm is cut off by the plate object among the infrared ray emitted from the infrared ray irradiator 130a. The cut-off infrared ray which wavelength is larger than 7.5 µm is once absorbed in the plate object and re-emitted from the plate object according to the temperature of the plate object. Some of them are re-emitted as the infrared ray which wavelength equals to or is smaller than 7.5 µm. Therefore, it is useful for enhancing the high infrared radiation efficiency.

It is possible for the plate object to give the directivity on the infrared ray. Fig. 3 is a schematic drawing showing the relationship between the depth (d) and the diameter (r) of the fine holes and the directivity of the reflected infrared ray. It is know that the directivity is given to the infrared ray by the relationship between the depth (d) and the diameter (r) of the fine holes installed on the surface of the ceramics 130a working as the irradiating material of the infrared ray. If the depth of the fine holes are larger than the diameter of the fine holes, the directivity of the emitted infrared ray is enhanced. By utilizing this characteristics, if there is certain distance from the non-contact type cell activation device of the present invention to the patient lesion (For example, in a range from dozens of centimeters to about over ten meters), the preferable amount of the infrared ray can be irradiated to the patient lesion. In the above-mentioned example, the ceramics is employed as the irradiator material, but other plate object such as rubber plate can be employed instead if the fine holes with the predetermined diameter can be installed on its surface.

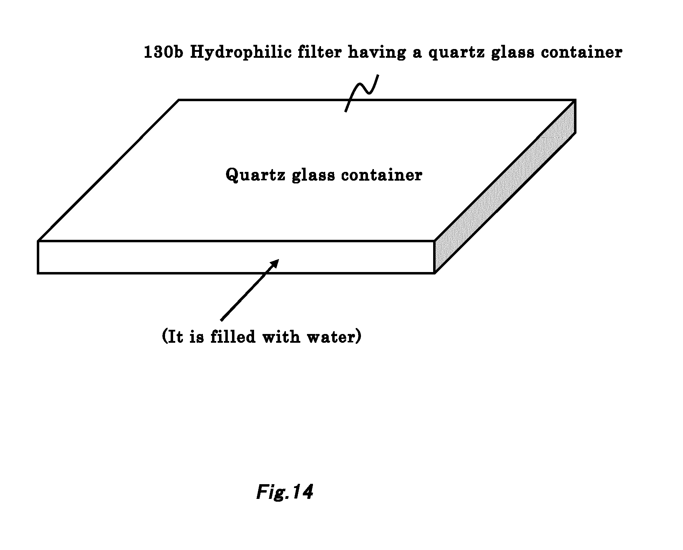

Next, a hydrophilic filter having a quartz glass container filled with water can be employed as an alternative of the optical filter. Fig.14 is a schematic drawing showing the water filter 130b filled with water in the quartz glass container. The spectral transmittance of a quartz glass for the infrared ray which wavelength is 1 to 10 µm is almost constant level. Therefore, the efficiency of the water filter 130b filled with water in the quartz glass container depends on the spectral transmittance of water. Water cuts off the infrared ray which wavelength is larger 7.5 µm passes through the infrared ray which wavelength equals to or is smaller than 7.5 µm. Therefore, the water filter 130b filled with water in the quartz glass container can be an alternative element of the optical filter 130 working as the infrared ray wavelength controller shown in embodiment 1. However, the water filter passes the infrared ray which wavelength is larger than 10 µm, so it is preferable that another optical filter which cuts off the infrared ray which wavelength is larger than 10 µm is employed together with the water filter 130b.

While some preferable embodiments of the cell activation device according to the present invention are described above, it should be understood that various changes are possible, without deviating from the technical scope according to the present invention. Therefore, the technical scope according to the present invention is limited only by the claims attached.

### Industrial applicability

The cell activation device according to the present invention can be used as the cell activation device. For example, it is used for assisting the cancer treatment, the cell activation device enabling a patient to receive cancer treatment while living an ordinary daily life.

## Claims

1. A cell activation device for irradiating infrared ray to a patient lesion including a cancer cell comprising;
a heat source;
a temperature controller;
an infrared ray irradiator;
wherein the infrared ray which wavelength equals to or is smaller than 7.5 µm is irradiated selectively from the infrared ray irradiator to the lesion cell by controlling the infrared ray wavelength emitted from the infrared ray irradiator by the temperature controller.

2. A cell activation device according to claim 1, further comprising;
a filter passing through the infrared ray which wavelength equals to or is smaller than 7.5 µm selectively installed onto the infrared ray irradiator,
wherein the infrared ray which wavelength is larger than 7.5 µm mainly working as the heat ray is cut off, and the infrared ray which wavelength equals to or is smaller than 7.5 µm is irradiated to the cell selectively.

3. A cell activation device according to claim 1 or 2, further comprising;
a fine holes are installed onto the surface of the infrared ray irradiator,
wherein the infrared ray irradiator does not generate the infrared ray which wavelength is larger than 7.5 µm mainly working as the heat ray by controlling the diameter of the fine holes is smaller than 15 µm;
and the infrared ray which wavelength equals to or is smaller than 7.5 µm is irradiated to the cell selectively.

4. A cell activation device according to claim 3, wherein the depth of the fine holes is controlled larger than the diameter of the fine holes installed on the surface of the infrared ray irradiator in order to obtain the radiation directivity on the infrared ray from the infrared ray irradiator.

5. A cell activation device according to claim 2, further comprising;
a first filter which is a hydrophilic filter having a quartz glass container filled with water and a second filter which can cut-off the infrared ray which wavelength is larger than 7.5 µm mainly working as heating ray.

6. A cell activation device according to any one of claims 1 to 5, wherein the temperature control part can maintain the temperature distribution of the heat source at a constant.

7. A cell activation device according to any one of claims 1 to 6, wherein the temperature control part can maintain the surface temperature of the infrared ray irradiator within the particular temperature range.
